# EUROPEAN PATENT SPECIFICATION

(11) **EP 1 482 968 B1**
(45) Date of publication and mention of the grant of the patent: **21.06.2006**
(21) Application number: 03711997.1
(22) Date of filing: 12.03.2003
(51) Int. Cl.: A61K 38/27

(54) **TREATMENT OF TYPE I DIABETES MELLITUS USING GROWTH HORMONE ANTAGONIST**
BEHANDLUNG VON TYP I DIABETES MELLITUS MIT WACHSTUMSHORMONANTAGONISTEN
TRAITEMENT DU DIABETE DE TYPE 1

(30) Priority: 13.03.2002 GB 0205898
(43) Date of publication of application: 08.12.2004
(73) Proprietor: Pharmacia & Upjohn Company LLC, Kalamazoo, MI 49001 (US)
(72) Inventor: DUNGER, David B., Cambridge Univ., Paediatr. Dept., Cambridge CB2 2QQ (GB); ACERINI, Carlo, Cambridge Univ., Paediatr. Dept., Cambridge CB2 2QQ (GB)
(74) Representative: Bannerman, David Gardner
(86) International application number: PCT/EP2003/002599
(87) International publication number: WO 2003/075946

(56) References cited:
- WO-A-95/35110
- WO-A-96/40203
- GOFFIN V ET AL: "PEGVISOMANT PHARMACIA" CURRENT OPINION IN INVESTIGATIONAL DRUGS, PHARMAPRESS, US, vol. 3, no. 5, 2002, pages 752-757, XP008019792 ISSN: 1472-4472
- JOURNAL OF CLINICAL ENDOCRINOLOGY AND METABOLISM, vol. 69, no. 2, 1989, pages 390-395, XP009016788
- ACTA PAEDIATRICA , vol. 85, no. 1, 1996, pages 31-37, XP009016831

## Description

Diabetes mellitus is a chronic metabolic disorder, which is brought about by either insulin deficiency or insulin resistance. Diabetes mellitus is a disease characterised by physiologic and anatomic abnormalities in many organs, due to vascular abnormalities. However, the most prominent feature of the disease is disturbed glucose metabolism, resulting in hyperglycaemia. Diabetes mellitus is usually divided into two major categories: insulin-dependent diabetes mellitus (Type I diabetes), which usually develops in childhood or adolescence and these patients are prone to ketosis and acidosis. The second category of patients (Type II diabetes) are not insulin dependent and usually manage with diet and oral hypoglycaemic therapy. The annual incidence of Type I diabetes ranges from 10 cases/100000 persons for non-white males to 16 cases/100000 persons for white males in the United States, with equal incidence between males and females. The prevalence of Type I diabetes for all ages in the United States population is 160 cases/100000 persons, with a slightly earlier onset for females with peak age of onset at 10-12 years than for males with peak age of onset at 18 years. Genetic background plays a major role in the development of the disease, with 40% concordance for Type I diabetes exhibited by identical twins and increased incidence among family members. Genes associated with increased susceptibility to Type I diabetes reside near the major histocompatibility complex on chromosome 6, with more than 90% of persons with Type I diabetes featuring DR3 or DR4 haplotypes or both. Likewise, siblings sharing DR3 or DR4 haplotypes from both parents more often than random develop Type I diabetes.

The onset of symptoms in Type I diabetes is usually acute and frequently follows an antecedent viral infection which might be the trigger to a process leading to destruction of the beta cells secondary to auto immune insulitis. When beta cell destruction reaches the critical point, the patient's reduced insulin levels lead to hyperglycaemia with the typical symptomatology of Type I diabetes. At diagnosis approximately 70% of patients with Type I diabetes have antibodies to islet cell cytoplasm i.e. antigens or to components of the islet cell surface. Approximately 15% of patients with Type I diabetes may also show other auto immune features, such as hypothyroidism, Graves' disease, Addison's disease, myasthenia gravis and pernicious anaemia. Autopsies of cases with Type I diabetes show a typical lymphocytic infiltration in the pancreatic islets.

Treatment of Type I diabetes at present is not satisfactory and the disease leads to serious life-threatening complications that can be only partly overcome with adequate control of insulin levels, which is usually difficult to accomplish in patients with juvenile onset. In addition to the acute diabetic syndrome, chronic manifestations lead to severe arteriosclerosis with microadenopathy affecting the eye with possible early blindness. One in 20 of all Type I diabetes patients becomes blind; about 40% of Type I diabetes develop renal failure, resulting in chronic hemodialisis and/or the need for renal transplantation (4-7). Severe neuropathic changes are also typical for Type I diabetes with many functional disorders associated with sensory, sympathetic and parasympathetic nerves. Cranial nerve, as well as peripheral nerve, may be involved. Treatment of neuropathy remains unsatisfactory, despite normal control of glucose levels with adequate insulin therapy.

Strokes are twice as frequent, myocardial infarctions are 2-5 times as frequent and cardiovascular accidents are 5-10 times more frequent in patients with Type I diabetes than among non-diabetic counterparts. The prognosis of patients with Type I diabetes who survive acute myocardial infarction is 3 times more grave compared to non-diabetics who survive acute infarction and the same is true for other vascular complications. Severe and uncontrollable arteriosclerosis may also be associated with a variety of etiologies involving abnormalities in platelets, clotting factors and lipid carriers, such as HGL levels, as well as uncontrolled diabetes.

The main treatment regime for Type I diabetes involves parental administration of insulin, usually subcutaneously. Insulin is destroyed in the gastrointestinal tract. A common regime for Type I diabetes patients is to inject a combination of short and intermediate acting insulins twice daily, before breakfast and before the evening meal. More intensive routines may involve multiple daily injections or continuous subcutaneous infusion of soluble insulin. The more intensive regimes tend to provide better control of blood glucose, however they are much more intrusive to the patient's life, which can be a particular problem when treating juveniles with this condition. Furthermore the intensive treatment regimes are more expensive.

There are several side effects associated with treatment with insulin, the most important being hypoglycaemia. This is a common side effect, particularly of the more intensive treatment regimes, which can result in severe morbidity and death. The symptoms include muscular weakness, incoordination, confusion and sweating. Severe hypoglycaemia can result in coma. Other side effects include allergy to insulin which may produce local or systemic reactions; loss or proliferation of fat at the sites of injection; and, rebound hyperglycaemia. Rebound hyperglycaemia usually occurs after an unrecognised hypoglycaemic attack, for example during sleep, and is caused by the release of counter-regulatory hormones in response to insulin-induced hypoglycaemia.

A particular problem is the so-called "dawn phenomenon" in which the blood sugar level rises in the early hours of the morning. Until now patients who suffer from this have been obliged to take insulin during the night, or risk suffering from night-time hypoglycaemia.

In view of the unsatisfactory prognosis for patients with Type I diabetes, and the side effects which may be experienced when using insulin to control the condition, it would be advantageous to have an alternative treatment which could be used instead of, or in combination with insulin. The inventors have considered the use of a growth hormone antagonist as a possible treatment for the condition.

Growth hormone (GH) is secreted by the anterior pituitary gland, under the control of the hypothalamus. It not only regulates growth, but also has metabolic effects, increasing protein synthesis, stimulating lipolysis, and increasing blood glucose. Its effects on carbohydrate metabolism are complex however. The somatogenic effects of GH are primarily mediated by insulin like growth factor-1 (IGF-1).

Insulin dependent diabetes causes profound derangement in the GH/IGF-1 axis. In poorly controlled Type 1 diabetics, GH levels are invariably raised. The elevated GH levels are characterised by a greater pulse amplitude and higher baseline concentration of GH as compared to the levels of normal subjects. Recent studies on the signal mode of GH indicate that it is the pulse amplitude rather than the increased baseline which lead to profound changes in insulin resistance in diabetic subjects (Pal et al., Diabetologia, in press). The high GH levels lead to insulin resistance and aggravate the metabolic abnormalities of diabetes. GH excess has also been implicated in the aetiology of the dawn syndrome and may accelerate the development of microangiopathy including proliferative retinopathy. Finally, an excessive rise in beta hydroxy buturate (BOH) caused by raised GH has been observed, particularly during puberty, and is compounded by the effects of insulin waning overnight; this leads to the risk of rapid decompensation with diabetic ketoacidosis in adolescents with diabetes.

Despite the elevated GH levels, IGF-I levels tend to be low in diabetes and this is related to decreased GH receptor function resulting from low levels of insulin (Holly J.M.P. et al., Clin Endocrinol, 29 (1988) 667-675). The lower IGF-I levels in the presence of elevated GH levels has been implicated in the slow growth and loss of adult height in children with diabetes (Salardi S. et al. Arch. Dis. Child, 62 (1987) 57-62).

The mechanism underlying the increased GH levels has been the subject of some controversy. In the diabetic individual hyperglycaemia does not inhibit GH secretion as it does in healthy individuals and it has been proposed that this reflects an altered hypothalamic function. This altered hypothalamic function is characterised by reduced somatostatin levels and resistance to the effects of somatostatin. Suppression of plasma GH by somatostatin analogues and pirenzepine has led to reported improvement in metabolic control. However, this approach has proved inappropriate during childhood and adolescence when growth is rapid as it would inevitably lead to growth failure.

The inventors have surprisingly found that administration of a growth hormone antagonist has a beneficial effect in patients suffering from Type I diabetes mellitus, especially in combatting the "dawn phenomenon" referred to above by reducing the patient's overnight insulin requirement.

The invention provides the use of growth hormone antagonists in the preparation of pharmaceutical compositions to reduce the overnight insulin requirement of a patient suffering from Type I diabetes mellitus. The pharmaceutical compositions of the invention may be used to treat mammalian species, in particular, humans.

The growth hormone antagonist is preferably pegvisomant (SOMAVERT ®).

Further provided by the invention is the above-mentioned use in a method of treating Type I diabetes mellitus comprising the step of administering insulin together with a growth hormone antagonist to a patient suffering from Type I diabetes mellitus during the evening in order to inhibit the dawn phenomenon. The patient may be a mammal, particularly a human.

The preferred growth hormone antagonist is pegvisomant (SOMAVERT ®). The method of treatment preferably comprises administering between 1mg and 20mg, more preferably between 5mg and 10mg of pegvisomant (SOMAVERT ®) once daily.

The invention will now be described in more detail by way of an example.

### Methods

7 adolescents of ages ranging from 16-23, the average age being 18, HbA₁₂ 10.0% (7.2-10.8) were randomized in a crossover study comparing pegvisomant (SOMAVERT ®) doses 5 mg and 10 mg once daily by subcutaneous injection at 18.00 hours. At baseline and after each 3-week treatment block subjects were admitted for an overnight variable rate insulin infusion (target glucose 5 m/mol/L) and two-step (0.75 and 1.5 ml⁵/Kg/min) hyperinsulinaemic euglycaemic clamp.

In more detail, prior to treatment and at the end of each treatment block, subjects underwent a fasted overnight (1800-0800h) variable rate insulin infusion for euglycaemia (5 mmol/l, achieved 0300 - 0800h) followed by a 2 step (0.75 and 1.5mU/kg/min) hyperinsulinaemic euglycaemic clamp (0800-1200h). Plasma insulin was measured every 30 min, - hydroxybutyrate (OHB) and free fatty acids (FFA) every 60 min. The stable isotopes ²H₂ glucose and ²H₅ glycerol were infused during the clamp to determine glucose and glycerol turnover respectively.

### Results

Data are expressed as mean (SEM) in the attached tables 1, 2 and 3. In the tables, * indicates p,0.05, **p 0.02 vs pre treatment values (P₀). IGF-I levels (ng/ml) fell with P₁₀: 154.8 28.1, ** vs P₀ 223.5 23.9. Overnight insulin requirements for euglycaemia (mU/kg/min) were reduced following treatment: P₅ 0.25 0.01*, P₁₀ 0.24 0.02 * * vs P₀ 0.34 0.03, whereas plasma glucose and insulin levels did not change. Overnight FFA (mmol/l): P₁₀ 0.38 0.04* vs P₀0.51 0.04 and OHB (mmol/l): P₁₀ 0.15 0.02** vs P₀0.31 0.04 were significantly reduced by P₁₀ only. There were no changes in endogenous hepatic glucose production but glucose disposal (mol/kg/min) fell during step 2 with P₅: 40.6 4.6* vs P₀ 48.5 4.2. Glycerol production (rate of appearance (Ra) (mol/kg/min) (n=5) was surpressed by P₁₀ during step 2: P₁₀ 1.2 0.1 * vs P₀ 2.0 0.3. In conclusion, GH blockade with pegvisomant results in reduction in overnight insulin requirements, with reduced glycerol Ra, and GH blockade with pegvisomant (10mg) results in reduction in FFA and OHB. Failure to observe corresponding changes in glucose turnover during the hyperinsulinaemic clamp may reflect reduction in circulating IGF-I. Results of further tests are shown in Table 4, as follows:

IGF-I levels (ngm/L) were reduced from 214.0 (26.4) to 207.1(34.9) after 5 mg pegvisomant (SOMAVERT ®) (P=0.7) and reduced to 144.2 (26.2) after 10 mg pegvisomant (SOMAVERT ®) (P=0.01).

Overnight (03.00-08.00 hours) insulin requirements for euglycaemia (mU/Kg/min) were 0.35(0.03) at baseline, 0.24 (0.04) after 5 mg pegvisomant (SOMAVERT ®) (P=0.02) and 0.25 (0.04) after 10 mg pegvisomant (SOMAVERT ®) (P=0.01).
Total body glucose disposal (m-value) was not altered during either step of the hyperinsulinaemic clamp (08.00-12.00 hours) by either dose of pegvisomant (SOMAVERT ®).

**TABLE 1 Fasting Bloods (0800h)**

| | Pre Treatment | 5mg | 10mg |
|---|---|---|---|
| IGF-I | 223.5 | 183.8 | 154.6* |
| | (23.9) | (38.1) | (28.1) |
| IGFBP-3 | 3018.3 | 2943.4 | 2689.6 |
| | (123.0) | (298.2) | (310.6) |

**TABLE 2 Overnight Steady State Period of Euglycaemia (0300-0800h)**

| | Pre-Treatment | 5mg | 10mg |
|---|---|---|---|
| Glucose | 5.3 | 5.1 | 5.5 |
| (mmol/l) | (0.07) | (0.04) | (0.09) |
| Insulin Requirement | 0.34 | 0.25* | 0.24** |
| (mU/Kg/min) | (0.03) | (0.01) | (0.02) |
| Plasma Insulin | 21.8 | 19.3 | 18.3 |
| (mU/I) | (2.4) | (1.4) | (1.6) |
| Metabolic Clearance Rate Insulin | 14.1 | 14.2 | 12.7 |
| | (1.5) | (1.1) | (1.3) |
| Non Essential Fatty Acid (NEFA) | 0.51 | 0.52 | 0.38* |
| (mmol/l) | (0.04) | (0.04) | (0.04) |
| β-Hydroxybutyrate | 0.31 | 0.25 | 0.15** |
| | (0.04) | (0.05) | (0.02) |
| IGFBP-1 | 44.3 | 56.6 | 53.0 |
| | (3.0) | (3.7) | (2.7) |

**TABLE 3 Hyperinsulinamic Clamp (0800-1200h)**

| | | Pre Treatment | 5mg | 10mg |
|---|---|---|---|---|
| Glucose | Step 1 | 4.9 | 5 | 4.9 |
| (mmol/l) | | (0.02) | (0.03) | (0.03) |
| | Step 2 | 5.0 | 5.0 | 4.9 |
| | | (0.06) | (0.06) | (0.04) |
| Plasma Insulin | Step 1 | 54.4 | 43.8 | 53.1 |
| (mU/l) | | (4.4) | (5.9) | (4.6) |
| | Step 2 | 97.9 | 98.4 | 107.4 |
| | | (5.3) | (12.6) | (7.2) |
| MCR-I | Step 1 | 15.0 | 17.0 | 14.9 |
| | | (1.3) | (1.6) | (1.4) |
| | Step 2 | 14.6 | 14.5 | 12.8 |
| | | (0.9) | (0.9) | (1.0) |
| m-value | Step 1 | 3.0 | 2.6 | 3.1 |
| (mg/kg/min) | | (0.4) | (0.3) | (0.5) |
| | Step 2 | 7.9 | 7.0* | 7.8 |
| | | (0.6) | (0.9) | (0.7) |
| Glucose Rd | Baseline | 9.5 | 10.1 | 9.6 |
| | | (1.1) | (0.6) | (0.8) |
| | Step 1 | 21.2 | 18.2 | 21.7 |
| | | (2.5) | (1.9) | (3.2) |
| | Step 2 | 48.5 | 40.6* | 46.7 |
| | | (4.2) | (4.6) | (5.8) |
| Glucose Ra | Baseline | 9.6 | 9.8 | 8.5 |
| | | (1.6) | (0.8) | (1.3) |
| | Step 1 | 4.1 | 3.6 | 2.6 |
| | | (0.9) | (0.6) | (0.9) |
| | Step 2 | 2.8 | 2.1 | 2.1 |
| | | (1.8) | (0.7) | (2.3) |
| Glycerol Ra | Baseline | 5.2 | 3.7 | 4.0 |
| (n=5) | | (0.9) | (0.4) | (0.3) |
| | Step 1 | 2.1 | 2.6 | 1.5 |
| | | (0.2) | (0.3) | (0.2) |
| | Step 2 | 1.7 | 2.5 | 1.1 * |
| | | (0.3) | (0.6) | (0.1) |

**Table 4**

| **Mean**^{**+**}**/- SEM** | **Baseline** | **5mg Treatment** | **10mg Treatment** | **Combined Treatment** |
|---|---|---|---|---|
| Fasting IGF-1 (ng/ml) | 214.15 ⁺/- 22.3 | 207.10⁺/- 34.9 | 144.24 ⁺/- 26.19 9 | 175.67⁺/- 22.69 |
| Overnight TBG (mmol/l) | 5.27⁺/- 0.07 | 5.01 ⁺/- 0.04 | 5.58 ⁺/- 0.09 | 5.29 ⁺/- 0.04 |
| Overnight Inuslin Requirement (mU/Kg/min) | 0.35 +/- 0.04 | 0.24 ⁺/- 0.01 | 0.25 ⁺/- 0.02 | 0.25 ⁺/- 0.01 1 |
| Overnight Plasma Insulin (mU/l) | 16.48 ⁺/- 1.1 | 19.24 ⁺/- 1.1 | 14.45 ⁺/- 1.1 | 16.67 ⁺/- 1.1 |

### Conclusions

Specific GH blockade reduced IGF-I levels and overnight insulin requirements. It did not affect insulin sensitivity the next morning, possibly because of the timing of the clamp or contracting effects of reductions in GH action and IGF-I on insulin sensitivity. Insulin requirements were reduced by around 31%.

## Claims

1. The use of the growth hormone (GH) antagonist pegvisomant (SOMAVERT®) in the preparation of a pharmaceutical composition to reduce the overnight insulin requirement of a patient suffering from Type 1 diabetes mellitus.

2. Use according to claim 1, wherein said composition is for administration by subcutaneous injection.

3. Use according to claim 1 or claim 2, wherein said composition is for daily administration.

4. Use according to claim 3, wherein said composition is for evening administration.

5. Use according to claim 1, wherein the composition is in the form of a daily dose of pegvisomant (SOMAVERT®) comprising from 1mg to 20mg.

6. Use according to claim 5, wherein the daily dose of pegvisomant is from 5mg to 10mg.

7. Use according to claim 6,w herein the daily dose of pegvisomant is 10mg.

8. Use according to claim 6, wherein the daily dose of pegvisomant is 5mg.

9. The use of the growth hormone (GH) antagonist pegvisomant (SOMAVERT®) in the preparation of a pharmaceutical composition to reduce the overnight insulin requirement of a patient suffering from dawn phenomenon.

## Patentansprüche

1. Verwendung des Wachtsumshormons(GH)-Antagonisten Pegvisomant (SOMAVERT® ) zur Herstellung einer pharmazeutischen Zusammensetzung zur Senkung des Insulinbedarfs eines an Diabetes mellitus Typ I leidenden Patienten während der Nacht.

2. Verwendung nach Anspruch 1, wobei die Zusammensetzung durch subcutane Injektion verabreicht wird.

3. Verwendung nach Anspruch 1 oder 2, wobei die Zusammensetzung täglich verabreicht wird.

4. Verwendung nach Anspruch 3, wobei die Zusammensetzung abends verabreicht wird.

5. Verwendung nach Anspruch 1, wobei die Zusammensetzung in Form einer täglichen Dosis Pegvisomant (SOMAVERT® ) von 1 mg bis 20 mg vorliegt.

6. Verwendung nach Anspruch 5, wobei die tägliche Dosis Pegvisomant 5 mg bis 10 mg beträgt

7. Verwendung nach Anspruch 6, wobei die tägliche Dosis Pegvisomant 10 mg beträgt.

8. Verwendung nach Anspruch 6, wobei die tägliche Dosis Pegvisomant 5 mg beträgt.

9. Verwendung des Wachstumshormons(GH)-Antagonisten Pegvisomant (SOMAVERT® ) zur Herstellung einer pharmazeutischen Zusammensetzung zur Senkung des Insulinbedarfs eines am Dawn-Phänomen leidenden Patienten über Nacht.

## Revendications

1. Application du pegvisomant ("SOMAVERT"), antagoniste de l'hormone de croissance (GH), à la préparation d'une composition pharmaceutique destinée à réduire la quantité d'insuline nécessaire pour la nuit à un patient souffrant de diabète "diabetes mellitus" de type 1.

2. Application selon la revendication 1, dans laquelle la composition est destinée à être administrée par injection sous-cutanée.

3. Application selon la revendication 1 ou 2, dans laquelle la composition est destinée à être administrée quotidiennement.

4. Application selon la revendication 3, dans laquelle la composition est destinée à être administrée le soir.

5. Application selon la revendication 1, dans laquelle la composition est sous la forme d'une dose quotidienne de pegvisomant ("SOMAVERT") contenant de 1 à 20 mg.

6. Application selon la revendication 5, dans laquelle la dose quotidienne de pegvisomant est comprise entre 5 et 10 mg.

7. Application selon la revendication 6, dans laquelle la dose quotidienne de pegvisomant est de 10 mg.

8. Application selon la revendication 6, dans laquelle la dose quotidienne de pegvisomant est de 5 mg.

9. Application du pegvisomant ("SOMAVERT"), antagoniste de l'hormone de croissance (GH), à la préparation d'une composition pharmaceutique destinée à réduire la quantité d'insuline nécessaire pour la nuit à un patient souffrant du phénomène de l'aube.
